# EUROPÄISCHE PATENTSCHRIFT

(11) **EP 1 390 745 B1**
(45) Veröffentlichungstag und Bekanntmachung des Hinweises auf die Patenterteilung: **24.08.2005**
(21) Anmeldenummer: 02750890.2
(22) Anmeldetag: 06.05.2002
(51) Int. Cl.: G01N 33/14

(54) **VERFAHREN ZUR KLASSIFIZIERUNG VON WEIN UND KAFFEE**
METHOD FOR CLASSIFYING WINE AND COFFEE
PROCEDE POUR CLASSER DU VIN ET DU CAFE

(30) Priorität: 28.05.2001 DE 10124917
(43) Veröffentlichungstag der Anmeldung: 25.02.2004
(73) Patentinhaber: Bionorica AG, 92318 Neumarkt (DE)
(72) Erfinder: POPP, Michael, A., Bionorica AG, 92318 Neumarkt (DE); BONN, Günther, Inst. für Analytische Chemie, 6020 Innsbruck (AT); HUCK, Christian, Inst. für Analytische Chemie, 6020 Innsbruck (AT); GUGGENBICHLER, W., Inst. für Analytische Chemie, 6020 Innsbruck (AT)
(74) Vertreter: Kaiser, Jürgen
(86) Internationale Anmeldenummer: PCT/EP2002/004988
(87) Internationale Veröffentlichungsnummer: WO 2002/097431

(56) Entgegenhaltungen:
- EP-A- 0 393 459
- WO-A-00/17611
- WO-A-89/09931
- FR-A- 2 797 688
- S. J. HASWELL: "multivariate data vvisualisation methods based on multi-elemental analysis of wine and coffees using total reflection X-ray fluorescence analysis" JOURNAL OF ANALYTICAL ATOMIC SPECTROMETRY, Bd. 13, Nr. 2, 1998, Seiten 131-134, XP008014386

## Beschreibung

Die vorliegende Erfindung betrifft ein Verfahren zur Klassifizierung von Getränken natürlicher Herkunft gemäß dem Oberbegriff des Anspruchs 1. Die Erfindung dient insbesondere der Klassifizierung von Wein und Kaffee.

Die Klassifizierung von Weinen nach beispielsweise Weinsorte, Weinanbaugebiet, Rebsorte und Jahrgang ist derzeit nur mit einem sehr gut trainierten Geruchs- und Geschmackssinn des Weinkenners auf sensorisch subjektivem Weg möglich. Neben den naturgegebenen Ungenauigkeiten, wie beispielsweise bei der Unterscheidung einzelner Jahrgänge eines Weines, sind diese sensorischen Qualitäten nur einem relativ kleinen Personenkreis vorbehalten.

Es hat daher in der Vergangenheit nicht an Versuchen gefehlt, die unterschiedlichen Weine mit wissenschaftlichen Methoden, etwa der Analyse einzelner chemischer Parameter, wie Zuckergehalt, Säuregrad, Ethanolgehalt usw. und/oder durch physikalisch chemische Methoden wie optische Rotationsdispersion, Brechungsindex, usw., zu untersuchen und durch die Interpretation der Einzeldaten, einer Gruppe von Daten oder der Gesamtheit dieser Daten eine eingangs genannte Klassifizierung zu bewerkstelligen. Siehe zum Beispiel FR-2 797 688.

Aufgrund der komplexen Zusammensetzung des Weines einerseits, jedoch der Ähnlichkeit einzelner Parameter andererseits sind bislang sämtliche Versuche einer verläßlichen Aussage mittels analytischer Verfahren über beispielsweise Weinsorte, Weinanbaugebiet, Rebsorte und Jahrgang eines in Rede stehenden Weines gescheitert.

Aus unterschiedlichen Gründen ist es jedoch sinnvoll, ein zuverlässiges Verfahren zur Klassifizierung von Weinen zur Verfügung zu haben. Einerseits ist so die lebensmitteltechnische Überwachung von Handelsprodukten und deren Übereinstimmung mit dem Weingesetz möglich, weil so beispielsweise erfaßt werden kann, ob die Kriterien der Nomination des Anbaugebietes erfüllt sind, ob also beispielsweise ein unzulässiger Verschnitt mit einer anderen Rebsorte/Anbaulage vorliegt. Andererseits wäre mit einem solchen Klassifizierungsverfahren auch die Steuerung des Herstellungsprozesses und Lagerreifung während der Weinherstellung durch den Winzer denkbar.

Neben dem oben erwähnten klassischen Stand der Technik der Weinanlytik ist es ferner aus der Diplomarbeit mit dem Titel "Anwendung multivariater Methoden und künstlicher neuronaler Netze zur Klassifizierung von Spirituosen mittels Headspace-GC/MS-Kopplung", vorgelegt von Patrick Kursawe, Lehrstuhl für analytische Chemie der Ruhr-Universität Bochum 1998, bekannt, unterschiedliche Schnäpse vom Grappa bis zum Rum unter Anwendung multivariater Methoden und künstlicher neuronaler Netze und Hauptkomponentenanalyse relativ zuverlässig zu klassifizieren.

In Bezug auf eine Weinklassifizierung mit den modernen chemometrischen Methoden haben Montanarella et al. [Montanarella, T., Bassani, M.R., Broas, O. (1995): Chemometric Classification of Some European Wines Using Pyrolysis Mass Spectrometry, Rapid Comm. Mass Spectrom. 9 (15), 1589-1593] versucht, unter Einsatz verschiedener multivariater Methoden sowie Backpropagation-Netze Weine bezüglich ihres Herkunftslandes anhand von Pyrolyse-Massenspektren zu klassifizieren. Eine feine Unterscheidung zwischen unterschiedlichen Regionen mißlang jedoch.

Ausgehend vom Stand der Technik der Montanarella et al. (1995) ist es daher Aufgabe der vorliegenden Erfindung, ein zuverlässiges Verfahren zur Klassifizierung von Getränken natürlicher Herkunft, insbesondere Weine und Kaffees - neben der visuell erkennbaren Farbe - mindestens nach der Wein- bzw. Kaffeesorte zu Verfügung zu stellen.

Die Aufgabe wird gelöst durch die kennzeichnenden Merkmale des Anspruchs 1.

Mit dem erfindungsgemäßen Verfahren ist es erstmals möglich eine Weinprobe wenigstens hinsichtlich ihrer zugehörigen Weinsorte (neben der visuell erkennbaren Farbe) zu klassifizieren. In der Regel sind jedoch sogar Klassifizierungen nach Rebsorte, Anbaugebiet und Jahrgang möglich.

Unter dem Begriff "Klasse" oder "Weinklasse" wird für die Zwecke der vorliegenden Erfindung eine Gruppe von Weinen mit definierten Eigenschaften, also Klasseneigenschaften, wie beispielsweise Weinsorte, Rebsorte, Anbaugebiet und Jahrgang, verstanden.

So kann beispielsweise die Weinklasse "Chianti Antinori 1996" die Klasseneigenschaften: Weinsorte: "Rotwein, Typ Chianti", "Rebsorte: Hauptbestandteil: Sangiovese" Anbaugebiet: "Südtirol" und Jahrgang "1996" aufweisen.

Mit dem erfindungsgemäßen Verfahren konnten mit unbekannten Weinproben beispielhaft bei folgenden Weinen eine eindeutige Klassifizierung erreicht werden:
Reinweine: Lagrein und Cabernet: Jahrgänge 1997 und 1998, Weinforschungszentrum Laimburg;
Chianti Classico Villa Antinori: Jahrgang 1996, Anbaugebiete Tirol und Südtirol, Rebsorte: Hauptbestandteil: Sangiovese;

Es hat sich ferner überraschend herausgestellt, daß sich das erfindungsgemäße Verfahren ebenfalls zur Klassifizierung von Kaffee nach den Klassen: Kaffesorte; Herkunftsland, Kaffeeanbaugebiet; Röstverfahren; chemische Parameter, insbesondere Coffeingehalt, Bitterstoffgehalt, Säuregrad, insbesondere Gehalt an Chlorogensäuren, toxikologische Parameter, insbesondere Herbizid- und Pestizidgehalt, besonders gut eignet.

Gemäß der vorliegenden Erfindung ist es bevorzugt, ein NIR-Spektrum der in Rede stehenden Weine ohne weitere Probenvorbereitung aufzunehmen. Hierfür kann beispielsweise ein handelsübliches NIR-VIS-Spektrometer verwendet werden. Die numerisch-mathematische Aufbereitung der Spektrenrohdaten kann mit einer ebenfalls handelsüblichen Software, z.B. BCAP V 6.00 [Firma BÜHLER AG, ANATEC, CH-9240 Uzwil, Schweiz] erfolgen.

Die Klassenkorrelation kann ebenfalls mit einer handelsüblichen Software, beispielsweise Nircal 3.0 [Firma Buchi AG, CH-9230 Flawil 1], z.B. mittels Hauptkomponentenanalyse und Clusterung erfolgen. Das Ergebnis kann beispielsweise in Form einer Clusterabbildung als 3D-Plot dargestellt werden, bei dem die Achsen die Hauptkomponenten darstellen.

Zur Kalibrierung des erfindungsgemäßen Verfahrens wird zunächst von einer Mehrzahl von hinsichtlich Weinsorte, Rebsorte oder Rebsorten, Anbaugebiet und Jahrgang bekannten Weinproben (in der Regel mindestens 10 Proben/Klasseneigenschaft) jeweils ein NIR-Spektrum , in der Regel mehrfach gemessen, um statistische Schwankungen abzupuffern. Diese Daten werden in der Regel numerisch-mathematisch aufbereitet, um die Datenmenge zu reduzieren und um sich auf die wesentlichen Charakteristiken der Spektren zu konzentrieren.

Dann wird das Verfahren mit diesen Proben derart korreliert, daß multivariate Methoden wie Hauptkomponentenanalyse, Clusterung, künstliche neuronale Netze auf diese aufbereiteten Daten angewendet wird, um aus der Flut der Daten eine Aussage treffen zu können, ob eine unbekannte Weinprobe, wenn sie ebenfalls NIR-spektroskopisch vermessen wird, zu dieser Klasse gehört oder nicht.

Unter multivariaten Methoden versteht man Auswerteverfahren, die mehr als nur ein Meßsignal derselben Probe verwenden, um zu einem Analysenergebnis zu kommen. Zu diesen Methoden zählen u.a. die multilineare Regression (MLR), Hauptkomponentenanalyse (PCA = Principal Components Analysis), Hauptkomponentenregression (PCR = Principal Components Regression), Methode der teilweisen kleinsten Quadrate (PLS: Partial Least Squares), Clustermethoden und künstliche neuronale Netze.

Für die künstlichen neuronalen Netze kommen insbesondere folgende Algorithmen in Betracht: Backpropagation-Netze, Dynamic Learning Vector Quantization (DLVQ-Algorithmus), Radial Basis Functions (RBF-Netze), insbesondere mit dem Dynamic Decay Adjustment Algorithmus (DDA-Algorithmus) trainierte RBF-Netze (RBF-DDA-Netz).

Die PCA führt eine Aufteilung der ursprünglichen Datenmatrix in zwei Matrizen durch, die Faktorenwerte und Ladungen (Loadings) genannt werden. Im ursprünglichen Datenraum wird ein Vektor so gewählt, daß bei einer Projektion der Daten auf ihn, der größtmögliche Teil der Varianz abgebildet wird. Dieser Vektor ist die erste Hauptkomponente. Orthogonal zur ersten Hauptkomponente wird eine zweite und gegebenenfalls eine zur ersten und zweiten Hauptkomponente orthogonal eine dritte, wobei die zweite und dritte Hauptkomponente möglichst viel der durch die ersten bzw. zweite Hauptkomponente noch nicht beschriebenen Varianz abbilden soll.

Die Koordinaten entlang der ersten Hauptkomponente enthalten die wesentlichen Informationen der Daten, die zweite und dritte Hauptkomponente geben im wesentlichen die Streuung wieder.

Dieser Vorgang wird wiederholt bis entweder die Zahl der Hauptkomponenten der der Dimension der Ausgangsdaten entspricht oder ein bestimmtes Abbruchkriterium erreicht wird.

Die so erhaltenen Hauptkomponenten sind Linearkombinationen der ursprünglichen Dimensionen. Sie sind linear unabhängig voneinander, wodurch eine definierte Anzahl von Hauptkomponenten weniger redundante Information enthält als die gleiche Anzahl der Ausgangsvariablen.

Ferner beschreiben die so erhaltenen Hauptkomponenten jeweils möglichst viel der Varianz der Ausgangsdaten, die von den vorliegenden Hauptkomponenten noch nicht beschrieben wurde. Dies führt dazu, daß in der Regel die ersten drei bis fünf Hauptkomponenten den wesentlichen Anteil der Information im Datensatz wiedergeben.

Mathematisch gesehen, ist die Hauptkomponentenanalyse ein Eigenwertproblem, dessen grundsätzliche Lösung dem Fachmann bekannt ist.

Das Resultat der Hauptkomponentenanalyse ist also eine Transformation des N-dimensionalen ursprünglichen Datenraumes, die zufolge hat, daß die ersten Dimensionen die wesentlichen, stark zur Gesamtvarianz beitragenden Datenteile enthalten und die letzten Dimensionen praktisch nur noch den Rauschanteil wiedergeben. Auf diese Weise ist die Struktur der in Rede stehenden spektroskopischen Daten durch Auftragung der ersten Hauptkomponenten gegeneinander darstellbar. Als zweidimensionale, vorzugsweise 3D-Abbildung stehen sie dann beispielsweise zur visuellen Auswertung durch den Anwender zur Verfügung, dem es überlassen bleibt eine solche Darstellung zu wählen, daß eine Einteilung von Weinproben in bestimmte Klassen erfolgen kann, was selbstverständlich auch automatisiert werden kann.

Bei der Kalibrierung können dann die sogenannten Toleranzkreise der Abbildungen so gewählt werden, daß sie an bestimmte Klassen bei Bedarf angepaßt werden können, um die Klassifizierung zu erleichtern.

Bevorzugt werden etwa 70% sämtlicher pro Klasse gemessener Weinproben zur Kalibrierung und etwa 30% zur Validierung des erfindungsgemäßen Verfahrens verwendet.

Zur besseren Reproduzierbarkeit des erfindungsgemäßen Verfahrens, werden die Proben bei einer konstanten Temperatur, vorzugsweise bei ca. 23°C, gemessen.

Mit dem erfindungsgemäßen Verfahren können Weinproben mit folgenden Klasseneigenschaften klassifiziert werden, wobei folgende Gruppe wenigstens teilweise umfaßt wird: Weinsorte; Weinanbaugebiet; Rebsorte; Rebwurzelstock; Jahrgang; Materialart, insbesondere Holzart des zur Lagerung/Reifung verwendeten Weinfasses, vorzugsweise Eichenart, z.B. amerikanische Eiche, Französische Eiche oder auch ungarische Eiche; unterschiedlicher Reifegrad der Lagerung im Faß; chemische Parameter, insbesondere Ethanolgehalt, Zuckergehalt, Säuregrad, SO₂-Gehalt; Tanningehalt; pH-Wert; Wassergehalt; Trockenrückstand; Polyphenolgehalt; toxikologische Parameter, insbesondere Glykolgehalt und/oder Methanolgehalt.

Weitere bevorzugte Ausführungsformen der vorliegenden Erfindung sind in den Unteransprüchen angegeben.

Ergänzende Vorteile und Merkmale der vorliegenden Erfindung ergeben sich aufgrund der Beschreibung von Ausführungsbeispielen sowie anhand der Zeichnung.

Es zeigt:
- Fig. 1: eine erste Clusterabbildung der Weinsorten: Chianti und Lagrein;
- Fig. 2: eine zweite Clusterabbildung der Weinsorten: Chianti und Lagrein;
- Fig. 3: eine erste Clusterabbildung der Weinsorten: Chianti, Lagrein und Cabernet;
- Fig. 4: eine zweite Clusterabbildung der Weinsorten: Chianti, Lagrein und Cabernet;
- Fig. 5: eine dritte Clusterabbildung der Weinsorten: Chianti, Lagrein und Cabernet;
- Fig. 6: eine Clusterabbildung der Cabernet-Jahrgänge 1997 und 1998; und
- Fig. 7: eine Clusterabbildung des Reifeprozesses.

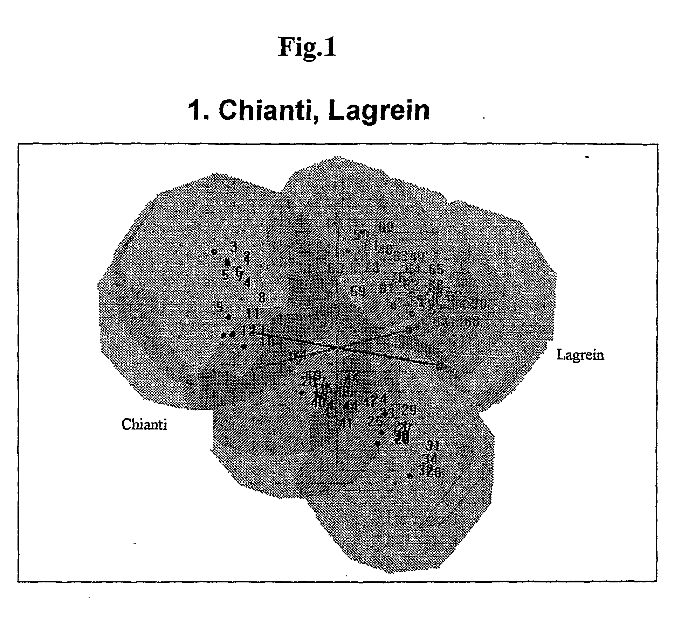

In den Fig. 1 bis 6 sind Clusterabbildungen für unterschiedliche Weine und Jahrgänge gezeigt.

Zunächst wurden von unterschiedlichen Weinen NIR-Spektren angefertigt. Diese Spektren wurden in 20 ml Meßküvetten ohne weitere Probenvorbereitung mit einem NIR-VIS-Spektrometer (FT-IR-Universalspektrometer) und mit der Software BCAP V6.0 (BÜHLER Analytical Package, BÜHLER AG, Anatec; CH-9240 Uzwil, Schweiz) bearbeitet.

Die Klassifizierung durch Hauptkomponentenanalyse/Clusterung erfolgte mittels der Software NIRCAL 3.0 (BÜHLER AG, Anatec; CH-9240 Uzwil, Schweiz). Hierbei handelt es sich um eine Software zur Steuerung des NIR-VIS-Spektrometers und chemometrischen Auswertung der aufgenommenen Spektren.

Die optischen Schichtdicken zur Messung der Spektren lag in den Beispielen bei 0,5 mm oder 3 mm.

Sämtliche Proben wurden bei einer konstant thermostatisierten Temperatur von ca. 23°C gemessen.

Die beispielhaft untersuchten Weine teilten sich auf drei unterschiedliche Weinklassen auf:
1. Reinweine, d.h. Weine, die zu 100% aus einer definierten Rebsorte hergestellt werden und aus einem einzigen (im Beispielsfalle relativ kleinem) Anbaugebiet stammen. In dieser Weinklasse wurden Weine der Rebsorte "Lagrein" vom Weinforschungszentrum Laimburg, Jahrgänge 1997 und 1998, verwendet.
2. Reinweine, d.h. Weine die zu 100% aus einer einzigen Rebsorte hergestellt werden und ebenfalls aus einem kleinen Anbaugebiet stammen. In dieser Weinklasse wurden Weine der Rebsorte "Cabernet" ebenfalls vom Weinforschungszentrum Laimburg, Jahrgänge 1997 und 1998, verwendet.
3. Weine, die aus einem breiten Anbaugebiet stammen und zu deren Herstellung mehrere Rebsorten verwendet werden. In dieser Weinklasse wurde ein "Chianti Antinori", Jahrgang 1996 verwendet, erworben in verschiedenen Geschäften in Tirol und Südtirol (Hauptbestandteil ist die Rebsorte "Sangiovese".
4. Als Qualitätskontrolle für das erfindungsgemäße Verfahren wurden mallorquinische Weine aus dem Weingut der Anmelderin verwendet.

Für die Kalibrierung der einzelnen Klasseneigenschaften wurden im Beispielsfalle jeweils wenigstens 15 Proben eingesetzt. Im Falle der Kalibrierung für einen Jahrgang wurden 10 Proben verwendet. Die Anzahl der Scans pro Spektrum und Probe lag zwischen 3 und 20.

Im 3D-Plot der drei Hauptkomonenten (Clusterabbildungen) war es möglich, die Weinklassen Lagrein, Sangiovese (Chianti) und Cabernet darzustellen. Unbekannte Proben konnten mittels des erfindungsgemäßen Verfahrens exakt klassifiziert werden.

Ferner ist es mit dem erfindungsgemäßen Verfahren möglich, zwischen den Jahrgängen 1997 und 1998 am Beispiel eines Cabernet-Weines zu unterscheiden und bei unbekannten Proben eine zuverlässige Aussage zu treffen, ob und zu welchen der beispielhaft angegebenen Jahrgänge diese zuzuordnen sind.

Im folgenden sind die Parameter angegeben, mit welchen die einzelnen 3D-Plots der Figuren 1 bis 6 aufgenommen wurden:
Fig. 1 zeigt eine erste Clusterabbildung der Weinsorten: Chianti und Lagrein, gemessen und ausgewertet mit folgenden Parametern:

| **Kalibrierungsprotokoll** | Schichtdicke: 0,5mm |
|---|---|
| Software | NIRCAL V3.04 (Build 216) |
| Verwendete Klassen im Kalibrierungssatz | chianti, lagrein. (total 2/2) |
| Gesamtzahl der Spektren | 1-81. (total 81/81) |
| Anzahl Kalibrierungsspektren | 51/81 |
| Anzahl Validierungsspektren | total 27/81 |
| Wellenlängenbereich [1/cm] | 4008-9996. (total 500/500) |
| Kalibrierungs-Wellenlängenbereich [1/cm] | 5628-7404. |
| Anzahl Rechenoperationen zur Datenvorbehandlung | 2 |
| Datenvorbehandlungssequenz | 1. Normalization between 0 to 1*, 5628-7404 |
| | 2. Second Derivative Taylor 3 Points |
| Chemometrisches Verfahren | Cluster |
| Anzahl der Primärfaktoren | 5 |
| Anzahl der Kalibrierungsfaktoren | 1-5. (total 5/5) |

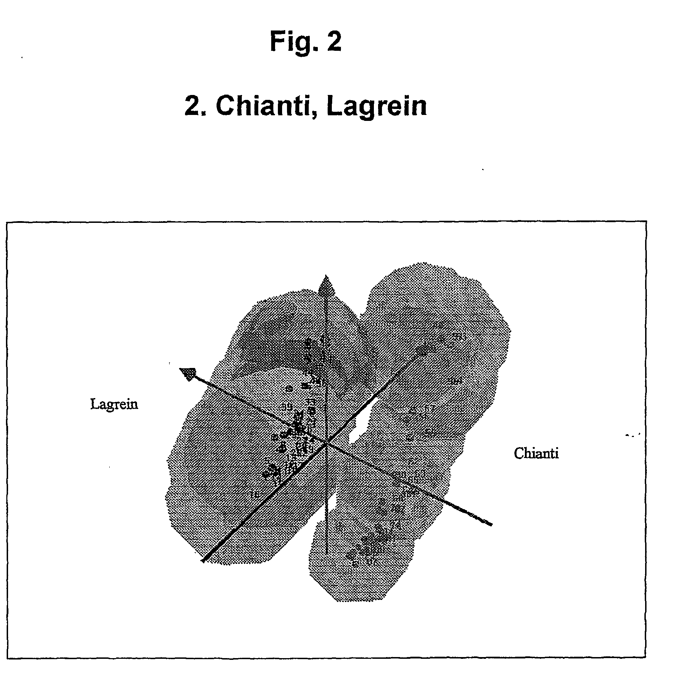

Fig. 2 zeigt eine zweite Clusterabbildung der Weinsorten: Chianti und Lagrein, gemesen und ausgewertet mit folgenden Parametern:

| **Kalibrierungsprotokoll** | Schichtdicke: 3mm |
|---|---|
| Properties in Project | lagrein, chianti. (total 2/2) |
| Verwendete Klassen im Kalibrierungssatz | lagrein, chianti. (total 2/2) |
| Gesamtzahl der Spektren | 1-91. (total 91/91) |
| Anzahl Kalibrierungsspektren | total 51/91 |
| Anzahl Validierungsspektren | total 40/91 |
| Kalibrierungs-Wellenlängenbereich [1/cm] | 4692-9960. |
| Anzahl Rechenoperationen zur Datenvorbehandlung | 2 |
| Datenvorbehandlungssequenz | 1. Normalization between 0 to 1*, 4692-9960 |
| | 2. Second Derivative Taylor 3 Points |
| Chemometrisches Verfahren | Cluster |
| Anzahl der Primärfaktoren | 4 |
| Anzahl der Kalibrierungsfaktoren | 1-3. (total 3/4) |

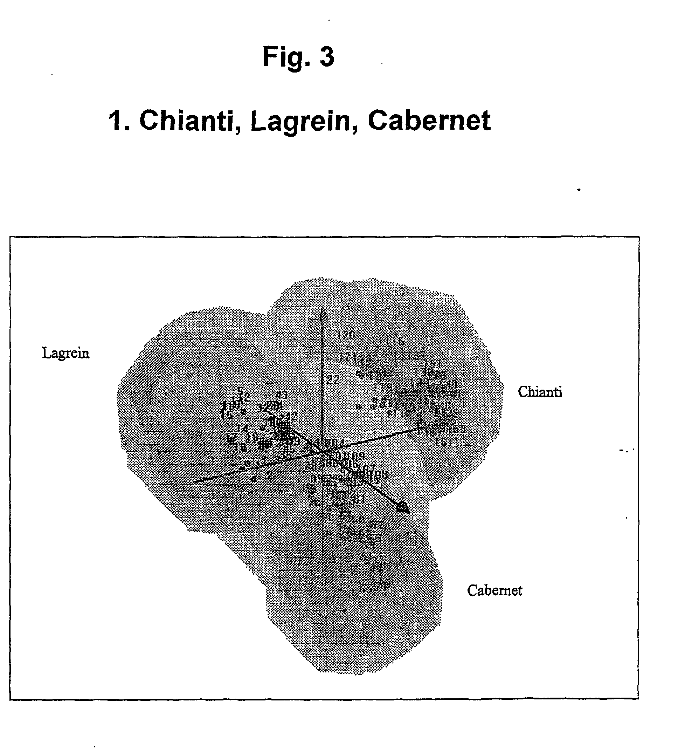

Fig. 3 zeigt eine erste Clusterabbildung der Weinsorten: Chianti, Lagrein und Cabernet, gemessen und ausgewertet mit folgenden Parametern:

| **Kalibrierungsprotokoll** | Schichtdicke: 3mm |
|---|---|
| Gesamtzahl der Spektren | 1-161. (total 161/161) |
| Anzahl Kalibrierungsspektren | total 116/161 |
| Anzahl Validierungsspektren | total 45/161 |
| Wellenlängenbereich [1/cm] | 4008-9996. (total 500/500) |
| Kalibrierungs-Wellenlängenbereich [1/cm] | 4428-9900. |
| Anzahl Rechenoperationen zur Datenvorbehandlung | 2 |
| Datenvorbehandlungssequenz | 1. Normalization by Maxima*, 4428-9900. (total 457/500) |
| | 2. Second Derivative Taylor 3 Points |
| Chemometrisches Verfahren | Cluster |
| Anzahl der Primärfaktoren | 6 |
| Anzahl der Kalibrierungsfaktoren | 1-5. (total 5/6) |

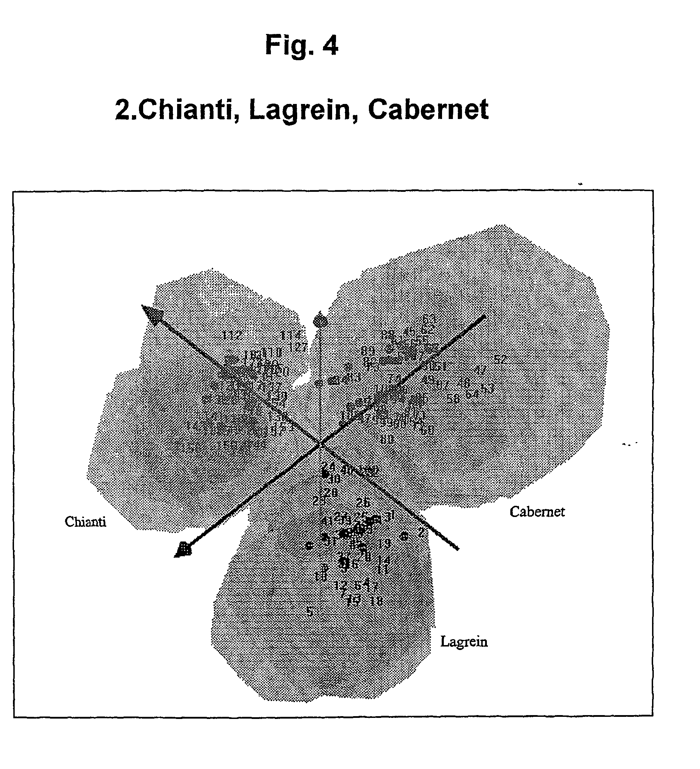

Fig. 4 zeigt eine zweite Clusterabbildung der Weinsorten: Chianti, Lagrein und Cabernet, gemessen und ausgewertet mit folgenden Parametern:

| **Calbration Protocol** | Schichtdicke: 3mm |
|---|---|
| Verwendete Klassen im Kalibrierungssatz | lagrein, cabernet, chianti. (total 3/3) |
| Gesamtzahl der Spektren | 1-157. (total 157/157) |
| Anzahl Kalibrierungsspektren | total 116/157 |
| Anzahl Validierungsspektren | total 41/157 |
| Wellenlängenbereich [1/cm] | 4008-9996. (total 500/500) |
| Kalibrierungs-Wellenlängenbereich [1/cm] | 4428-9900. |
| Anzahl Rechenoperationen zur Datenvorbehandlung | 2 |
| Datenvorbehandlungssequenz | 1. Smooth Average 3 Points |
| | 2. Second Derivative Taylor 3 Points |
| Chemometrisches Verfahren | Cluster |
| Anzahl der Primärfaktoren | 4 |
| Anzahl der Kalibrierungsfaktoren | 1-3. (total 3/4) |

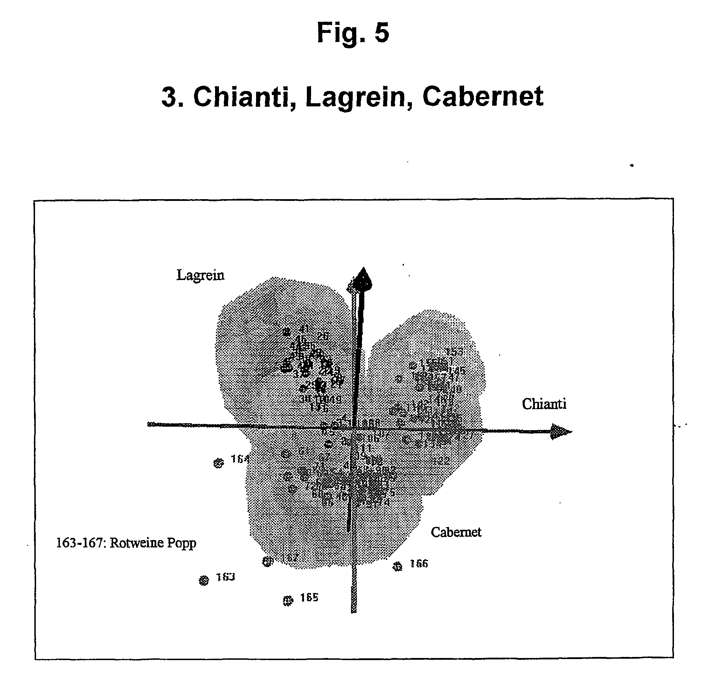

Fig. 5 zeigt eine dritte Clusterabbildung der Weinsorten: Chianti, Lagrein und Cabernet, gemessen und ausgewertet mit folgenden Parametern:

| **Kalibrierungsprotokoll** | Schichtdicke 3mm |
|---|---|
| Verwendete Klassen im Kalibrierungssatz | lagrein, cabernet, chianti. (total 3/3) |
| Anzahl Kalibrierungsspektren | total 119/167 |
| Anzahl Validierungsspektren | total 48/167 |
| Spectra unused (U-Set) | nothing selected. (total 0/167) |
| Wellenlängenbereich [1/cm] | 4008-9996. (total 500/500) |
| Kalibrierungs-Wellenlängenbereich [1/cm] | 4428-9900. |
| Anzahl Rechenoperationen zur Datenvorbehandlung | 2 |
| Datenvorbehandlungssequenz | 1. Smooth Average 3 Points |
| | 2. Second Derivative Taylor 3 Points |
| Chemometrisches Verfahren | Cluster |
| Anzahl der Primärfaktoren | 5 |
| Anzahl der Kalibrierungsfaktoren | 1-5. (total 5/5) |

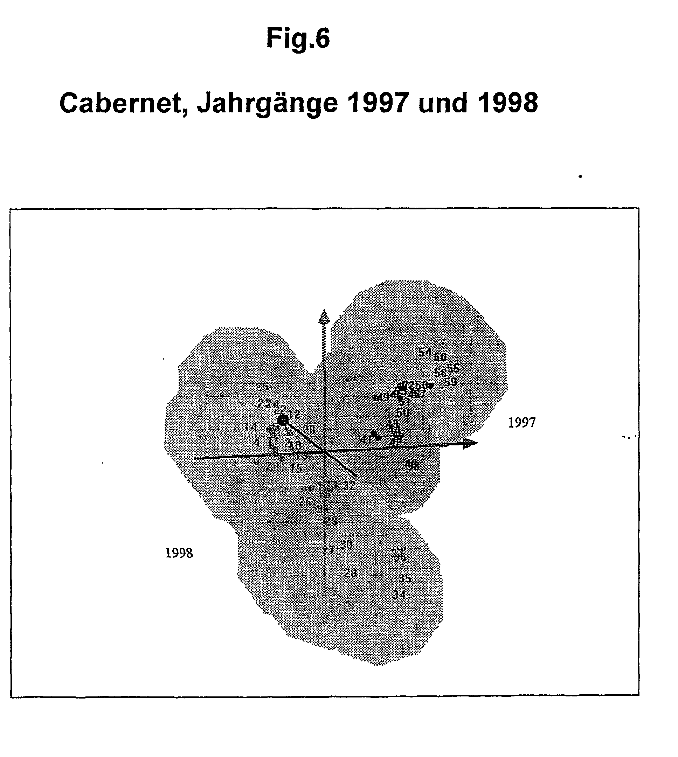

Fig. 6 zeigt eine Clusterabbildung der Cabernet-Jahrgänge 1997 und 1998, gemessen und ausgewertet mit folgenden Parametern:

| **Kalibrierungsprotokoll** | Schichtdicke: 3mm |
|---|---|
| Verwendete Klassen im Kalibrierungssatz | 97, 98. (total 2/2) |
| Gesamtzahl der Spektren | 1-60. (total 60/60) |
| Anzahl Kalibrierungsspektren | total 45/60 |
| Anzahl Validierungsspektren | total 15/60 |
| Wellenlängenbereich [1/cm] | 4008-9996. (total 500/500) |
| Kalibrierungs-Wellenlängenbereich [1/cm] | 4512-9996. |
| Anzahl Rechenoperationen zur Datenvorbehandlung | 2 |
| Datenvorbehandlungssequenz | 1. Smooth Average 3 Points |
| | 2. Second Derivative Taylor 3 Points |
| | |
| Chemometrisches Verfahren | Cluster |
| Anzahl der Primärfaktoren | 3 |
| Anzahl der Kalibrierungsfaktoren | 1-3. (total 3/3) |

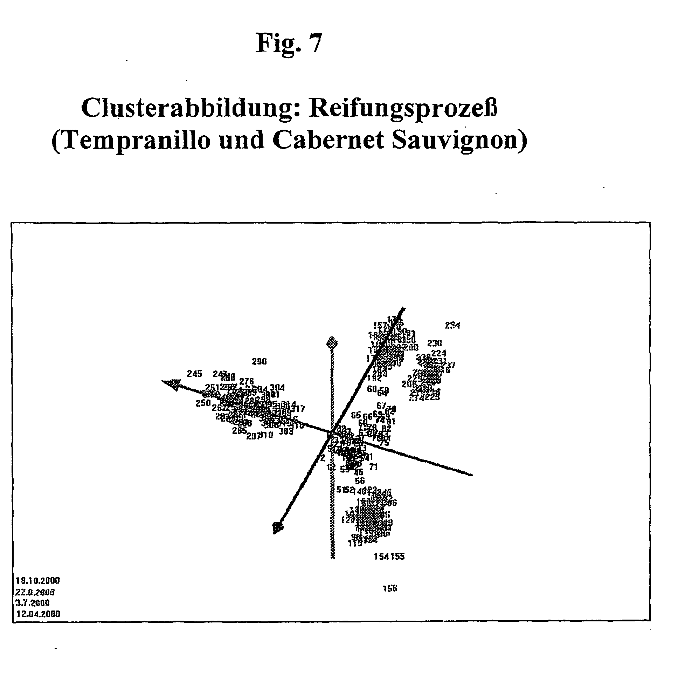

Fig. 7 zeigt eine Clusterbildung des Reifeprozesses von Weinen der Rebsorte Tempranillo und Cabernet Sauvignon, gemessen und ausgewertet mit folgenden Parametern:

| **Kalibrierungsprotokoll** | Schichtdicke: 3mm |
|---|---|
| Verwendete Klassen im | 12.04., 3.7., 22.8. und |
| Kalibrierungssatz | 18.10.2000 (total 4/7) |
| Gesamtzahl der Spektren | Total 213/318 |
| Anzahl Kalibrierungsspektren | total 105/318 |
| Anzahl Validierungsspektren | total 15/60 |
| Wellenlängenbereich [1/cm] | 4008-9996. (total 500/500) |
| Kalibrierungs-Wellenlängenbereich [1/cm] | 4512-9996. |
| Anzahl Rechenoperationen zur Datenvorbehandlung | 2 |
| Datenvorbehandlungssequenz | 1. Normalisierung zwischen 0 bis 1*, 4500-9996 (total 459/500) |
| | 2. Erste Ableitung Derivative Taylor 3 Punkte |
| | |
| Chemometrisches Verfahren | Cluster |
| Anzahl der Primärfaktoren | 8 |
| Anzahl der Kalibrierungsfaktoren | 1-4. (total 4/8) |

Die in den obigen Tabellen aufgelisteten Parameter - sofern nicht selbsterklärend - haben ferner folgende Bedeutung :
Kalibrierungsprotokoll: Schichtdicke 0,5 mm: Die für die Kalibrierung verwendete optische Schichtdicke beträgt 0,5 mm.
Anzahl Rechenoperationen zur Datenvorbehandlung: Es handelt sich um die Anzahl der mathematischen Rechenoperationen zur Vorbehandlung der Spektren.

## Patentansprüche

1. Verfahren zur Klassifizierung von Getränken natürlicher Herkunft, welches folgende Schritte umfaßt:
a) Bereitstellen einer Mehrzahl von Getränkeklassen mit jeweils einer Mehrzahl von Kalibrierungs-Getränkeproben pro Klasse mit einer Mehrzahl bekannter Klasseneigenschaften, wobei die Klasseneigenschaften der einzelnen Weinklassen mehrere Eigenschaften aus folgender Gruppe sind: Weinsorte; Weinanbaugebiet; Rebsorte; Rebwurzelstock; Jahrgang; Materialart, insbesondere Holzart des zur Lagerung/Reifung verwendeten Weinfasses, vorzugsweise Eichenart, z.B. amerikanische Eiche, Französische Eiche, ungarische Eiche; oder Mischformen der Hölzer; unterschiedlicher Reifegrad der Lagerung im Faß; chemische Parameter, insbesondere Ethanolgehalt, Zuckergehalt, Säuregrad, SO2-Gehalt; Tanningehalt; pH-Wert; Wassergehalt; Trockenrückstand; Polyphenolgehalt; toxikologische Parameter, insbesondere Glykolgehalt und/oder Methanolgehalt; und wobei
die Klasseneigenschaften der einzelnen Kaffeeklassen mehrere Eigenschaften aus folgender Gruppe sind: Kaffesorte; Herkunftsland; Kaffeeanbaugebiet; Röstverfahren; chemische Parameter, insbesondere Coffeingehalt, Bitterstoffgehalt, Säuregrad, insbesondere Gehalt an Chlorogensäuren;
b) Einstrahlen von Meßlicht aus einem vorgegebenen Wellenlängenbereich in die Getränkeproben;
c) Erfassen des durch die Getränkeproben hindurch tretenden, reflektierten, reemitierten und/oder gestreuten Meßlichts;
d) Bestimmen des wellenlängenabhängigen Verhältnisses von eingestrahltem zu erfaßtem Meßlicht (Spektrum) für jede Getränkeprobe einer Klasse, wobei ein Wellenlängenbereich im NIR-VIS-Bereich liegt;
e) numerisch-mathematische Aufbereitung der Spektraldaten der einzelnen Getränkeproben;
f) Korrelation der Spektren einer Mehrzahl von Getränkeproben mit einer vorgegebenen Getränkeklasse;
g) Erstellen einer Datenbank aus den aufbereiteten Spektraldaten mit unterschiedlichen Getränkeklassen, basierend auf den gemessenen Getränkeproben der einzelnen Klassen zur Kalibrierung einer Klassenkorrelation;
h) Wenigstens einmaliges Wiederholen der Schritte b) bis e) mit wenigstens einer Getränkeprobe mit wenigstens teilweise unbekannten Eigenschaften; und
i) Bestimmen der Getränkeklassen, zu welcher die unbekannte Getränkeprobe zuzuordnen ist, mittels einer Klassenkorrelation der gemessenen Spektren unter Verwendung der erstellten Kalibrierungsdatenbank aus Schritt g);
j) wobei die Korrelation der numerisch-mathematisch aufbereiteten Spektraldaten mittels einer Clusterbildung erfolgt.

2. Verfahren nach Anspruch 1, **dadurch gekennzeichnet, daß** als Getränk Wein verwendet wird.

3. Verfahren nach Anspruch 1, **dadurch gekennzeichnet, daß** als Getränk Kaffe verwendet wird.

4. Verfahren nach einem der Ansprüche 1 bis 3, **dadurch gekennzeichnet, daß** der Wellenlängenbereich im Bereich von 700 bis 2 500 nm, vorzugsweise im Bereich von 1 000 bis 2 200 nm (4 500 bis 10 000 cm-1), liegt.

5. Verfahren nach einem der Ansprüche 1 bis 4, **dadurch gekennzeichnet, daß** das Meßlicht mittels eines Lichtwellenleiters durch die Getränkeproben eingeleitet und/oder aus ihnen aufgenommen wird.

6. Verfahren nach einem der Ansprüche 1 bis 5, **dadurch gekennzeichnet, daß** die optische Schichtdicke der Probe zwischen 0,2 und 5 mm, vorzugsweise bei 0,5 oder 3 mm liegt.

7. Verfahren nach einem der Ansprüche 1 bis 6, **dadurch gekennzeichnet, daß** die Getränkeproben zur Messung thermostatisiert werden, vorzugsweise auf 23°C.

8. Verfahren nach einem der Ansprüche 1 bis 7, **dadurch gekennzeichnet, daß** die numerisch-mathematische Aufbereitung eine Datenreduktion umfaßt, insbesondere durch: Normalisierung, Glättung, 1. Ableitung, 2. Ableitung, Multiplicative Scatter Correction, Kehrwert, Quadrat, Mean Centering, Kubelka Munc Transformation, Absorption, Basislinienkorrektur, Addition einer Konstanten, Shift Negative to Zero.

9. Verfahren nach einem der Ansprüche 1 bis 8, **dadurch gekennzeichnet, daß** die Korrelation der numerisch-mathematisch aufbereiteten Spektraldaten multivariate Methoden, vorzugsweise eine Hauptkomponentenanalyse (PCA) und/oder eine Glättung und/oder wenigstens eine Reihenentwicklung, insbesondere eine Taylorreihenentwicklung und/oder künstliche neuronale Netzalgorithmen, insbesondere Backpropagation-Netze, Dynamic Learning Vector Quantization (DLVQ-Algorithmus), Radial Basis Functions (RBF-Netze), insbesondere mit dem Dynamic Decay Adjustment Algorithmus (DDA-Algorithmus) trainierte RBF-Netze (RBF-DDA-Netz); umfaßt.

10. Verfahren nach einem der Ansprüche 1 bis 9, **dadurch gekennzeichnet, daß** die Toleranzkreise der einzelnen Cluster bei der Kalibrierung einstellbar sind.

11. Verfahren nach einem der Ansprüche 1 bis 10, **dadurch gekennzeichnet, daß** 3 bis 20 Spektral-Scans/Getränkeprobe aufgenommen werden.

12. Verfahren nach einem der Ansprüche 1 bis 11, **dadurch gekennzeichnet, daß** wenigstens 10 Getränkeproben pro Klasseneigenschaft zur Kalibrierung verwendet werden.

13. Verfahren nach einem der Ansprüche 1 bis 12, **dadurch gekennzeichnet, daß** etwa 70% sämtlicher pro Klasse gemessenen Getränkeproben zur Kalibrierung und etwa 30% zur Validierung des Verfahrens verwendet werden.

14. Verfahren nach einem der Ansprüche 1 bis 13, **dadurch gekennzeichnet, daß** das Klassifizierungsergebnis als 3D-Plot auf einem Bildschirm dargestellt wird.

## Claims

1. Method for classifying beverages of natural origin, including the following steps:
a) providing a plurality of beverage classes, with a plurality of calibration beverage samples per class each, having a plurality of known class properties, wherein the class properties of the individual wine classes are several properties from the following group: sort of wine; growing region; grape; vine; vintage; kind of material, in particular species of wood of the wine cask used for storage/maturing, preferably kind of oak, e.g., American oak, French oak, Hungarian oak; or mixed forms of woods; various degree of maturity of storage in cask; chemical parameters, in particular ethanol content, sugar content, acidity, SO₂ content; tannin content; pH value; water content; dry residue; polyphenol content; toxicological parameters, in particular glycol content and/or methanol content; and wherein
the class properties of the individual coffee classes are several properties from the following group: coffee sort; country of origin; coffee growing region; roasting method; chemical parameters, in particular coffein content, bittering content, acidity, in particular chlorogenic acids content;
b) irradiating measurement light from a predetermined wavelength range into the beverage samples;
c) detecting the measurement light passed through, reflected, re-emitted, and/or dispersed from, the beverage samples;
d) determining the wavelength-dependent ratio of irradiated to detected measurement light (spectrum) for each beverage sample of a class;
e) numerical-mathematical conditioning of the spectral data of the individual beverage samples;
f) correlating the spectra of a plurality of beverage samples with a predetermined beverage class;
g) compiling a database from the conditioned spectral data with different beverage classes based on the measured beverage samples of the individual classes for calibration of a class correlation;
h) repeating at least once the steps b) to e) with at least one beverage sample having at least partly unknown properties; and
i) determining the beverage classes to which the unknown beverage sample is to be associated, with the aid of a class correlation of the measured spectra, by using the compiled calibration database of step g);
j) wherein correlation is performed by cluster formation.

2. Method according to claim 1, **characterized in that** wine is used as the beverage.

3. Method according to claim 1, **characterized in that** coffee is used as the beverage.

4. Method according to any one of claims 1 to 3, **characterized in that** the wavelength range is in the range of approx. 700 to 2,500 nm, preferably in the range of approx. 1,000 to 2,200 nm (4,500 to 10,000 cm⁻¹).

5. Method according to any one of claims 1 to 4, **characterized in that** the measurement light is introduced through the beverage samples and/or received from them with the aid of a light waveguide.

6. Method according to any one of claims 1 to 5, **characterized in that** the optical layer thickness of the sample is between about 0.2 and 5 mm, preferably at approx. 0.5 or approx. 3 mm.

7. Method according to any one of claims 1 to 6, **characterized in that** the beverage samples are thermostated for measurement, preferably at approx. 23°C.

8. Method according to any one of claims 1 to 7, **characterized in that** numerical-mathematical conditioning encompasses a data reduction, in particular by: normalization, smoothing, 1st derivation, 2nd derivation, multiplicative scatter correction, reciprocal value, square, mean centering, Kubelka Munc transformation, absorption, baseline correction, addition of a constant, shift negative to zero.

9. Method according to any one of claims 1 to 8, **characterized in that** correlation of numerically-mathematically conditioned spectral data encompasses multivariate methods, preferably a principal components analysis (PCA) and/or a smoothing and/or at least one series development, in particular a Taylor series development and/or artificial neuronal network algorithms, in particular backpropagation networks, dynamic learning vector quantization (DLVQ algorithm), radial basis functions (RBF networks), in particular RBF networks (RBF-DDA network) trained with the dynamic decay adjustment algorithm (DDA algorithm).

10. Method according to any one of claims 1 to 9, **characterized in that** the tolerance circles of the individual clusters are adjustable in calibration.

11. Method according to any one of claims 1 to 10, **characterized in that** approx. 3 to 20 spectral scans/beverage sample are recorded.

12. Method according to any one of claims 1 to 11, **characterized in that** at least approx. 10 beverage samples per class property are used for calibration.

13. Method according to any one of claims 1 to 12, **characterized in that** about 70% of all beverage samples measured per class are used for calibration, and about 30% for validation of the method.

14. Method according to any one of claims 1 to 13, **characterized in that** the classification result is represented on a screen as a 3-D plot.

## Revendications

1. Procédé pour la classification de boissons d'origine naturelle, qui comprend les étapes suivantes :
a) Mise à disposition d'une pluralité de classes de boissons avec respectivement une pluralité d'échantillons de boisson de calibrage par classe avec une pluralité de propriétés connues de classe, sachant que les propriétés de classe des différentes classes de vin sont plusieurs propriétés de la groupe suivante:
sorte de vin ; vignoble ; cépage ; pied de vigne ; année ; sorte de matériau, en particulier sorte de bois du tonneau utilisé pour le stockage/la maturation, de préférence type de chêne, par exemple chêne d'Amérique, chêne de France, chêne de Hongrie ;
ou formes mélangées de bois ; degré de maturation différent de stockage dans le tonneau ; paramètres chimiques, en particulier teneur en éthanol, teneur en sucre, degré d'acidité, teneur en SO² ; teneur en tanin ; valeur PH ; teneur en eau ; résidu sec ; teneur en polyphénol ; paramètres toxicologiques, en particulier teneur en glycol et/ou teneur en méthanol ; et sachant que les propriétés de classe des différentes classes de café sont plusieurs propriétés de la groupe suivante:
sorte de café ; pays d'origine ; région de culture ; procédé de torréfaction ; paramètres chimiques, en particulier teneur en caféine, teneur en substance amère, degré d'acidité, en particulier teneur en acides chlorogéniques ;
b) Projection d'un rayon lumineux de mesure à partir d'une zone de longueur d'onde prescrite dans les échantillons de boisson ;
c) Saisie du rayon lumineux de mesure traversant les échantillons de boisson, réfléchi, réémi et/ou dispersé ;
d) Détermination du rapport dépendant de la longueur d'onde du rayon lumineux de mesure projeté au rayon lumineux de mesure saisi (spectre) pour chaque échantillon de boisson d'une classe, sachant qu'une zone de longueur d'onde se situe dans la zone NIR-VIS ;
e) Préparation numérique-mathématique des données spectrales des différents échantillons de boisson ;
f) Corrélation des spectres d'une pluralité d'échantillons de boisson avec une classe de boisson donnée ;
g) Création d'une banque de données à partir des données spectrales préparées avec différentes classes de boisson, prenant comme base les échantillons de boisson mesurés des différentes classes pour le calibrage d'une corrélation de classe ;
h) Répétition au moins une fois des étapes b) à e) avec au moins un échantillon de boisson avec au moins en partie des propriétés inconnues ; et
i) Détermination de la classe de boisson, à laquelle doit être affecté l'échantillon de boisson inconnu, au moyen d'une corrélation de classe des spectres mesurés en utilisant la banque de données de calibrage créée à l'étape g) ;
j) Sachant que la corrélation des données spectrales préparées de manière numérique-mathématique s'effectue au moyen d'une formation d'amas.

2. Procédé selon la revendication 1, **caractérisé en ce que** du vin est utilisé comme boisson.

3. Procédé selon la revendication 1, **caractérisé en ce que** du café est utilisé comme boisson.

4. Procédé selon l'une quelconque des revendications 1 à 3, **caractérisé en ce que** la zone de longueur d'onde se situe dans la plage de 700 à 2 500 nm, de préférence dans la plage de 1 000 à 2 200 nm (4 500 à 10 000 cm-1).

5. Procédé selon l'une quelconque des revendications 1 à 4, **caractérisé en ce que** le rayon lumineux de mesure est conduit à travers les échantillons de boisson et/ou réceptionné à partir de ceux-ci au moyen d'un conducteur d'onde lumineuse.

6. Procédé selon l'une quelconque des revendications 1 à 5, **caractérisé en ce que** l'épaisseur de couche optique de l'échantillon se situe entre 0,2 et 5 mm, de préférence à 0,5 ou 3 mm.

7. Procédé selon l'une quelconque des revendications 1 à 6, **caractérisé en ce que** les échantillons de boisson sont thermostatés pour la mesure, de préférence à 23°C.

8. Procédé selon l'une quelconque des revendications 1 à 7, **caractérisé en ce que** la préparation numérique-mathématique comprend une réduction de données, en particulier par : normalisation, lissage, 1^{ère} dérivation, 2^{ème} dérivation, multiplicative scatter correction, valeur inverse, carré, mean centering, Kubelka Munc transformation, absorption, correction de ligne de base, addition d'une constante, shift negative to zero.

9. Procédé selon l'une quelconque des revendications 1 à 8, **caractérisé en ce que** la corrélation des données spectrales préparées de manière numérique-mathématique comprend des méthodes multivariables, de préférence une analyse des composants principaux (PCA) et/ou un lissage et/ou au moins un développement en série, en particulier un développement en série de Taylor et/ou des algorithmes de réseau neuronaux artificiels, en particulier des réseaux de rétropropagation, de dynamic learning vector quantization (algorithme DLVQ), radial basis functions (réseaux RBF), en particulier des réseaux RBF (réseau RBF-DDA) entraînés avec un algorithme dynamic decay adjustement (algorithme DDA).

10. Procédé selon l'une quelconque des revendications 1 à 9, **caractérisé en ce que** les cercles de tolérance des différents amas peuvent être réglés pendant le calibrage.

11. Procédé selon l'une quelconque des revendications 1 à 10, **caractérisé en ce que** sont pris 3 à 20 scans spectraux par échantillon de boisson.

12. Procédé selon l'une quelconque des revendications 1 à 11, **caractérisé en ce que** sont utilisés pour le calibrage au moins 10 échantillons de boisson par propriété de classe.

13. Procédé selon l'une quelconque des revendications 1 à 12, **caractérisé en ce qu'**environ 70 % de tous les échantillons de boisson mesurés par classe sont utilisés pour le calibrage et environ 30 % pour la validation du procédé.

14. Procédé selon l'une quelconque des revendications 1 à 13, **caractérisé en ce que** le résultat de la classification est représenté comme tracé 3D sur un écran.
